# EUROPEAN PATENT APPLICATION

(11) **EP 4 317 452 A1**
(43) Date of publication of application: **07.02.2024**
(21) Application number: 22774634.4
(22) Date of filing: 25.01.2022
(51) Int. Cl.: C12Q 1/06, A23L 33/12, A61K 31/20, A61K 31/23, A61K 45/00, A61P 1/18, A61P 3/04, A61P 3/10, A61P 25/28, A61P 43/00, G01N 33/15, G01N 33/50

(54) **SCREENING METHOD FOR GLP-1 SECRETION CONTROL AGENT, GLP-1 SECRETION CONTROL AGENT, COMPOSITION FOR PREVENTING OR AMELIORATING DIABETES, OBESITY, POSTPRANIDAL HYPERGLYCEMIA, NEURODEGENERATIVE DISEASE, HYPOGLYCEMIA, ADIPOSITY, OR NESIDIOBLASTOSIS, METHOD FOR PROMOTING SECRETION OF GLP-1 IN GPR84-EXPRESSING CELLS, METHOD FOR PRODUCING COMPOSITION FOR PROMOTING SECRETION OF GLP-1, USE OF GPR84 AGONIST FOR PRODUCING THERAPEUTIC AGENT FOR DISEASE, GLP-1 SECRETION PROMOTION AGENT, AND COMPOSITION FOR ORAL INGESTION**

(30) Priority: 22.03.2021 JP 2021047568
(71) Applicant: THE NISSHIN OILLIO GROUP, LTD., Tokyo 104-8285 (JP)
(72) Inventor: KIMURA Ikuo, Fuchu-shi, Tokyo 183-8538 (JP); OHUE Ryuji, Fuchu-shi, Tokyo 183-8538 (JP); IGARASHI Miki, Fuchu-shi, Tokyo 183-8538 (JP); WATANABE Shinji, Yokohama-shi, Kanagawa 235-8558 (JP); YAMAUCHI Akiko, Yokohama-shi, Kanagawa 235-8558 (JP)
(74) Representative: KATZAROV S.A.
(86) International application number: PCT/JP2022/002650
(87) International publication number: WO 2022/201832

(57) **Abstract**

The present invention addresses the problem of providing a novel GLP-1 secretion promotion agent. The present invention provides a GLP-1 secretion promotion agent that includes a GPR84 agonist as an active ingredient.

## Description

### TECHNICAL FIELD

The present invention relates to a screening method for a GLP-1 secretion regulation agent, a GLP-1 secretion regulation agent, a composition for preventing or ameliorating diabetes, obesity, postprandial hyperglycemia, neurodegenerative diseases, hypoglycemia, adiposity, or nesidioblastosis, a method for promoting secretion of GLP-1 in GPR84-expressing cells, a method for producing a composition for promoting secretion of GLP-1, use of a GPR84 agonist for producing therapeutic agent for disease, a GLP-1 secretion promotion agent, and a composition for oral ingestion.

### BACKGROUND ART

In recent years, the increase in the number of diabetic patients in Japan has been of concern. Actually, the market of antidiabetic drugs is the second largest in Japan after anticancer drugs (in the fiscal year 2019).

Various antidiabetic drugs have been developed, and examples thereof include a drug capable of promoting secretion of Glucagon-like peptide-1 (hereinafter, also referred to as "GLP-1"), and the like.

GLP-1 is one of the gastrointestinal hormones, which is secreted from the L cells of the small intestine as a result of increase in blood glucose level due to food ingestion, and the like. The secreted GLP-1 binds to the GLP-1 receptor on pancreatic β cells and allows insulin to be secreted from the β cells. Therefore, GLP-1 promotes insulin secretion, resulting in normalizing blood glucose levels, so that diabetes can be treated.

Various substances functioning as the GLP-1 secretion promotion agent are known (see, for example, Patent Documents 1 and 2).

### Citation List

### Patent Document

Patent Document 1: Japanese Unexamined Patent Application, Publication No. 2013-63956
Patent Document 2: Japanese Unexamined Patent Application, Publication No. 2016-138070

### DISCLOSURE OF THE INVENTION

### Problems to be Solved by the Invention

However, there are still needs for a novel GLP-1 secretion promotion agent.

The present invention has been made in view of the situation mentioned above, and has an object to provide a novel GLP-1 secretion promotion agent.

### Means for Solving the Problems

The present inventors have newly found that the ligand of "GPR84", which is a G protein-coupled receptor (hereinafter also referred to as "GPCR"), has a function of promoting GLP-1 secretion via binding to GPR84, and have completed the present invention. More specifically, the present invention provides the following.

(1) A screening method for a GLP-1 secretion regulation agent, the method including:
   a first measurement step of bringing a test substance into contact with GPR84-expressing cells, and measuring an amount of GLP-1 secretion from the GPR84-expressing cells;
   a second measurement step of measuring an amount of GLP-1 secretion from the GPR84-expressing cells under the same conditions as in the first measurement step except that the test substance is not brought into contact with GPR84-expressing cells; and
   a comparison step of comparing a measurement result of the first measurement step with a measurement result of the second measurement step,
   wherein, in the comparison step, when a measured value of the first measurement step is higher or lower than a measured value of the second measurement step, the test substance is identified as a GLP-1 secretion regulation agent.
(2) The screening method according to (1), wherein in the first measurement step, the test substance is brought into contact with the GPR84-expressing cells in a presence of medium chain fatty acid.
(3) A GLP-1 secretion regulation agent, being identified by the screening method according to (1) or (2).
(4) A composition for prevention or amelioration of diabetes, obesity, postprandial hyperglycemia, neurodegenerative diseases, hypoglycemia, adiposity, or nesidioblastosis, the composition including the GLP-1 secretion regulation agent according to (3).
(5) A method for promoting secretion of GLP-1 in GPR84-expressing cells, the method including bringing a GPR84 agonist into contact with GPR84-expressing cells.
(6) A production method of a composition for promoting secretion of GLP-1, the method including:
   a contact step of bringing a GPR84 agonist candidate substance into contact with GPR84-expressing cells in vitro;
   a measurement step of measuring an amount of GLP-1 secretion from the GPR84-expressing cells before and after the contact step; and
   an identification step of identifying a GPR84 agonist candidate substance, in which an amount of GLP-1 secretion after the contact step is increased to more than an amount of GLP-1 secretion before the contact step, as a GLP-1 secretion promotion agent.
(7) A production method of a composition for promoting secretion of GLP-1, the method including:
   an administration step of administering a GPR84 agonist candidate substance to a mammal (excluding humans);
   a sample obtaining step of obtaining a biological sample from the mammal before and after the administration step;
   a measurement step of measuring an amount of GLP-1 in the biological sample obtained before and after the administration step; and
   an identification step of identifying a GPR84 agonist candidate substance, in which an amount of GLP-1 secretion after the administration step is increased to more than an amount of GLP-1 secretion before the administration step, as a GLP-1 secretion promotion agent.
(8) The production method according to (7), wherein the biological sample is blood.
(9) The production method according to any one of (6) to (8), further including a composition preparation step of preparing a composition including the GLP-1 secretion promotion agent identified in the identification step and a pharmaceutically acceptable carrier.
(10) The production method according to any one of (6) to (9), further including a structure analysis step of analyzing a structure of the GPR84 agonist candidate substance after the identification step.
(11) The production method according to any one of (6) to (10), further including a synthesis step of synthesizing the GPR84 agonist candidate substance after the identification step.
(12) The production method according to any one of (6) to (11), wherein the amount of GLP-1 secretion is an amount of biologically active GLP-1.
(13) The production method according to any one of (6) to (12), wherein the GPR84 agonist candidate substance is a human GPR84 agonist.
(14) The production method according to any one of (6) to (13), wherein the GPR84 agonist candidate substance is an orally administrable substance.
(15) The production method according to any one of (6) to (14), wherein the GPR84 agonist candidate substance is a selective GPR84 agonist.
(16) The production method according (15), wherein the selective GPR84 agonist candidate substance has EC₅₀ of less than 10 µM.
(17) The production method according to any one of (6) to (16), wherein the GPR84 agonist candidate substance is a low-molecular-weight compound.
(18) The production method according to any one of (6) to (17), wherein the GPR84 agonist candidate substance is medium chain fatty acid.
(19) Use of a GPR84 agonist for producing a therapeutic agent for disease, wherein the disease is one or more diseases selected from the group consisting of diabetes, obesity, postprandial hyperglycemia, and neurodegenerative diseases.
(20) A GLP-1 secretion promotion agent including a GPR84 agonist as an active component.
(21) The GLP-1 secretion promotion agent according to (20), wherein the GPR84 agonist is medium chain fatty acid.
(22) The GLP-1 secretion promotion agent according to (21), wherein a number of carbon atoms of the medium chain fatty acid is 9 to 12.
(23) The GLP-1 secretion promotion agent according to (21) or (22), wherein the medium chain fatty acid is in a form of constituent fatty acid of triacylglycerol and/or free fatty acid.
(24) The GLP-1 secretion promotion agent according to (21), wherein the medium chain fatty acid is in a form of triacylglycerol, and
   the triacylglycerol is one or more triacylglycerol selected from the group consisting of triacylglycerol including only medium chain fatty acid having 10 carbon atoms as the constituent fatty acid, and triacylglycerol including medium chain fatty acid having 8 carbon atoms and medium chain fatty acid having 10 carbon atoms as the constituent fatty acid.
(25) The GLP-1 secretion promotion agent according to (24), wherein the triacylglycerol is triacylglycerol including medium chain fatty acid having 8 carbon atoms and medium chain fatty acid having 10 carbon atoms as the constituent fatty acid, and
   a mass ratio of the medium chain fatty acid having 8 carbon atoms to the medium chain fatty acid having 10 carbon atoms satisfies: the medium chain fatty acid having 8 carbon atoms:the medium chain fatty acid having 10 carbon atoms = 30:70 to 75:25.
(26) The GLP-1 secretion promotion agent according to any one of (20) to (25), further including an active component being other than the GPR84 agonist and including a GLP-1 secretion promotion effect.
(27) The GLP-1 secretion promotion agent according to any one of (20) to (26), which are administered in a fasted state.
(28) The GLP-1 secretion promotion agent according to any one of (20) to (27), which is to be used for one or more purposes selected from the group consisting of:
   preventing or ameliorating diabetes, obesity, postprandial hyperglycemia, and neurodegenerative diseases;
   ameliorating neuropathy associated with diabetes;
   suppressing glucagon secretion;
   suppressing gastric excretion and/or gastrointestinal secretions;
   suppressing appetite and/or food ingestion; and promoting proliferation of pancreatic β cells.
(29) A composition for prevention or amelioration of diabetes, obesity, postprandial hyperglycemia, or neurodegenerative disease, the composition including a GLP-1 secretion promotion agent according to any one of (20) to (28) .
(30) The composition for prevention or amelioration according to (29), being in a form of a drug, a quasi-drug, or food.
(31) A composition for oral ingestion, including a GLP-1 secretion promotion agent according to any one of (20) to (28) .
(32) The composition for oral ingestion according to (31), being administered so that 1 g or more per day of medium chain fatty acid is ingested.
(33) The composition for oral ingestion according to (31) or (32), being administered in a fasted state.
(34) The composition for oral ingestion according to any one of (31) to (33), being in a form of a beverage, seasoning, or other food.

### Effects of the Invention

The present invention provides a new GLP-1 secretion promotion agent.

### BRIEF DESCRIPTION OF THE DRAWINGS

Fig. 1 is a view showing an effect of medium chain fatty acid on GLP-1 secretion;
Fig. 2 is a view showing an effect of medium chain fatty acid on insulin secretion;
Fig. 3 is a view showing an effect of medium chain fatty acid on GLP-1 secretion; and
Fig. 4 is a view showing an effect of triacylglycerol on GLP-1 secretion.

### PREFERRED MODE FOR CARRYING OUT THE INVENTION

Hereinafter, embodiments of the present invention are described, but the present invention is not limited thereto.

### <GLP-1 secretion promotion agent>

A GLP-1 secretion promotion agent of the present invention includes a GPR84 agonist as an active component.

As mentioned above, although various agents have been known as the GLP-1 secretion promotion agent, the present inventors have firstly found that GPR84, which has not been known in terms of functions at all, is related to the secretion of GLP-1. In other words, the present inventors have firstly specified that the secretion of GLP-1 is promoted via bonding between a GPR84 agonist and GPR84. Therefore, in the future, GPR84 could be a promising development target of antidiabetic drugs.

Conventionally, GPR84 was known to be highly expressed in the brain, pituitary gland, bone marrow, and the like, and to be activated by medium chain fatty acid and the like. However, the downstream signal of GPR84 has not been clarified so far, and the function of GPR84 remained unknown. Therefore, the above findings have great significance in that the function of GPR84 was elucidated for the first time. In particular, since an expression amount of GPR84 is not so large in the digestive tract (small intestine, and the like), it has never been assumed that GPR84 is related to digestion or metabolism. Therefore, it is quite surprising that GPR84 is greatly involved in the secretion of GPL-1 as the gastrointestinal hormone. Therefore, in a preferable embodiment of the present invention, "GPR84" includes GPR84 expressed in cells expressing in the gastrointestinal tract (especially small intestine endocrine cells).

In the present invention, the "GLP-1 secretion promotion agent" refers to an agent capable of increasing the amount of GLP-1 secreted in vivo when the secretion promotion agent is administered, as compared with a case where the secretion promotion agent is not administered. In the present invention, the "GLP-1 secretion promotion effect" means that when a certain substance (GPR84 agonist in the present invention) is administered, the amount of GLP-1 secreted in vivo is increased as compared with a case where the substance is not administered. The amount of GLP-1 secretion in vivo can be determined using a commercially available GLP-1 ELISA kit or the like.

In the present invention, the "GPR84 agonist" refers to a substance capable of being bonded to G protein-coupled receptor 84 (GPR84) and activating an intracellular signaling system similar to in vivo substances. Whether a substance is a GPR84 agonist or not can be identified by the screening method of the present invention, which will be described later.

In the present invention, the "active component" of a GLP-1 secretion promotion agent refers to a physiologically active substance having a GLP-1 secretion promotion effect.

### (Example of GPR84 agonist)

Examples of the GPR84 agonist are not particularly limited, and examples thereof include medium chain fatty acid (hereinafter, also referred to as "MCFA"), Embelin, 6-OAU(6-n-octylaminouracil), DIM(3,3'-diindolylmethane), and the like. The GPR84 agonist may be used singly or in combination of two or more. Among the above, from the viewpoint that the advantageous effect of the present invention is easily obtained, as the GPR84 agonist, medium chain fatty acid is preferable.

MCFA is straight-chain saturated fatty acid having 6 to 12 carbon atoms, and is a fat component included in ordinary foods (for example, edible oils and fats, dairy products, and the like). Therefore, when the GPR84 agonist is medium-chain fatty acid, a highly safe GLP-1 secretion promotion agent is likely to be obtained.

The number of carbon atoms in MCFA is preferably 9 to 12, more preferably 10 or 12, and most preferably 10, from the viewpoint of achieving a high GLP-1 secretion promoting effect. When the number of carbon atoms in MCFA is 10, in a case where the MCFA is in the form of triacylglycerol, the triacylglycerol becomes liquid at human body temperature and is easily absorbed.

Specific examples of MCFA include caproic acid (n-hexanoic acid), caprylic acid (n-octanoic acid), capric acid (n-decanoic acid), and lauric acid. Among these, capric acid (n-decanoic acid) and lauric acid are preferred.

MCFA can be obtained, for example, by refining after hydrolyzing palm kernel oil or coconut oil. Commercially available products and reagents can also be used as MCFA.

The form of MCFA is not particularly limited, and may be a medium chain fatty acid itself (free fatty acid, a free form), or a fatty acid precursor that is to be converted into a medium chain fatty acid in vivo (for example, salt, ester (acylglycerol, and the like, mentioned later)), or a mixture thereof. In the present invention, MCFA is preferably in the form of a constituent fatty acid of acylglycerol (especially, triacylglycerol) and/or free fatty acid.

MCFA is usually ingested into the body in the form of a fatty acid precursor, more specifically, acylglycerol in which MCFA and glycerin are ester-bonded. It is known that ingested acylglycerol is decomposed and absorbed in the digestive tract, releases MCFA, and is converted into energy in the liver.

Acylglycerol has a structure in which fatty acid and glycerin are ester-bonded, and exists in one of three forms (monoacylglycerol, diacylglycerol, and triacylglycerol) depending on the number of fatty acids bound to glycerin. The acylglycerol in the present invention may be in any of the above three forms, but at least one of its constituent fatty acids should be MCFA. Note here that triacylglycerol in which all constituent fatty acids are MCFA is also called medium chain triglyceride (MCT).

As the acylglycerol in the present invention, triacylglycerol is preferable from the viewpoint of being close to the usual food form. Furthermore, in the present invention, fatty acids constituting diacylglycerol and triacylglycerol may be of the same type or of different types. In the case of acylglycerol composed of different types of fatty acids, the bonding position of each fatty acid to glycerin is not particularly limited. Furthermore, the constituent fatty acid of acylglycerol may include fatty acids other than MCFA (for example, long chain fatty acids having 14 to 22 carbon atoms, and the like).

When MCFA is in the form of triacylglycerol, it is preferable that the triacylglycerol is one or more triacylglycerol selected from the group consisting of triacylglycerol only including medium chain fatty acid having 10 carbon atoms as constituent fatty acids, and triacylglycerol including medium chain fatty acid having 8 carbon atoms and medium chain fatty acid having 10 carbon atoms as constituent fatty acids.

When MCFA is in the form of triacylglycerol, it is preferable that the triacylglycerol is triacylglycerol including medium chain fatty acid having 8 carbon atoms and medium chain fatty acid having 10 carbon atoms as the constituent fatty acid, and that a mass ratio of the medium chain fatty acid having 8 carbon atoms to medium chain fatty acid having 10 carbon atoms satisfies medium chain fatty acid having 8 carbon atoms : medium chain fatty acid having 10 carbon atoms = 30 : 70 to 75 : 25. When the mass ratio of the medium chain fatty acid having 8 carbon atoms to the medium chain fatty acid having 10 carbon atoms is in the above range, the effect of promoting the GLP-1 secretion can be obtained more easily.

The lower limit of the proportion of MCFA in the entire constituent fatty acids of acylglycerol is preferably 10% by mass or more, more preferably 50% by mass or more, and further more preferably 70% by mass or more. The upper limit is preferably 100% by mass or less. When the MCFA ratio is within the above range, the effect of promoting GLP-1 secretion is more likely to be obtained.

The production method of acylglycerol is not particularly limited, but acylglycerol can be obtained, for example, by subjecting MCFA derived from palm kernel oil or coconut oil to esterification reaction with glycerin. Examples of the esterification reaction include a method of reacting under pressure without a catalyst and a solvent, a method of reacting using a synthetic catalyst such as sodium methoxide, and a method of reacting using lipase as a catalyst.

The content of MCFA contained in the GLP-1 secretion promotion agent of the present invention can be identified by gas chromatography.

### (Other components)

The GLP-1 secretion promotion agent of the present invention may consist of a GPR84 agonist, but may include other components together with the GPR84 agonist. Other components are not particularly limited as long as they do not inhibit the action of the GPR84 agonist. Such components include active components other than GPR84 agonist, and antioxidants, emulsifiers, long chain fatty acid triglycerides, and the like.

As the active component other than the GPR84 agonist, an active component having a GLP-1 secretion promotion effect is preferable from the viewpoint that the effects of the present invention are more likely to be exhibited. Such components include glucose, amino acids, and the like.

The lower limit of the amount of the GPR84 agonist contained in the GLP-1 secretion promotion agent of the present invention is preferably 10% by mass or more, more preferably 50% by mass or more with respect to the entire GLP-1 secretion promotion agent. The upper limit is preferably 100% by mass or less, more preferably 70% by mass or less. When the blending amount of the GPR84 agonist is within the above range, the effect of promoting GLP-1 secretion is more likely to be obtained.

### (Usage of GLP-1 secretion promotion agent, and the like)

The GLP-1 secretion promotion agent of the present invention can be used in any subjects or setting where the concentration of GLP-1 is to be increased.

For example, the GLP-1 secretion promotion agent of the present invention may be administered in a fasted state. By administering the GLP-1 secretion promotion agent of the present invention to a subject in a fasted state, it is possible to prevent a rapid increase in the blood glucose level or the like during food ingestion after administration.

The "fasted state" of the present invention refers to a time point when digestion progresses and blood glucose level decrease and lipolysis occur after food ingestion, and includes, for example, 1 to 3 hours after food ingestion.

For example, the GLP-1 secretion promotion agent of the present invention may be used for any one or more of the following purposes.
Preventing or ameliorating diabetes, obesity, postprandial hyperglycemia, and neurodegenerative diseases
Ameliorating neuropathy associated with diabetes
Suppressing glucagon secretion
Suppressing gastric excretion and/or gastrointestinal secretions
Suppressing appetite and/or food ingestion
Promoting proliferation of pancreatic β cells

The "prevention" in the present invention refers to, for example, suppressing or delaying the onset of a disease or a symptom.

The "amelioration" in the present invention refers to, for example, delaying the progression of a disease or a symptom, and relief, reduction, amelioration, cure, or the like, of a symptom.

The "diabetes" in the present invention includes both type 1 diabetes and type 2 diabetes.

The "obesity" in the present invention refers to a state in which fat is excessively accumulated in adipose tissue in vivo, and includes, for example, a body mass index (BMI) of 25 or more.

The "postprandial hyperglycemia" in the present invention refers to an abnormal increase in blood glucose level after food ingestion, and includes, for example, a state in which the blood glucose level continues to be 140 mg/dl or more after 2 to 3 hours after food ingestion.

The "neurodegenerative disease" in the present invention refers to a neurodegenerative disease associated with abnormal insulin secretion, and examples of the neurodegenerative disease include Alzheimer's disease, Parkinson's disease, Huntington's disease, and the like.

The "diabetic neuropathy" in the present invention refers to, for example, complications that cause abnormalities in the nerves of the hands and feet due to hyperglycemia, resulting in sensory abnormalities such as pain and numbness in the tips and soles of the feet and fingers of the hand.

The "suppressing glucagon secretion" in the present invention refers to a decrease in blood glucagon concentration due to the GLP-1 secretion promotion agent of the present invention.

The "suppression of gastric excretion" in the present invention refers to suppression or delay of excretion of food digested in the stomach into the duodenum.

The "suppression of gastrointestinal secretion" in the present invention refers to adjusting the amount of secretion of enzymes and hormones for digestion and absorption of foods (for example, reducing the amount of secretion).

The "suppressing appetite and/or food ingestion" in the present invention refers to a decrease in appetite and/or food ingestion amount due to administration of the GLP-1 secretion promotion agent of the present invention.

The "promoting proliferation of pancreatic β cells" in the present invention refers to an increase in production of pancreatic β cells by administration of the GLP-1 secretion promotion agent of the present invention.

The administration target of the GLP-1 secretion promotion agent of the present invention is not particularly limited. Examples thereof include any isolated cell, any isolated tissue, or living body. There is no particular limitation on the origin of cells or tissues, or the type of living organism, and may be mammals (humans, mice, rats, rabbits, cows, dogs, cats, etc.), fish, reptiles, and the like.

### <Composition including GLP-1 secretion promotion agent of the present invention>

As described above, the GLP-1 secretion promotion agent of the present invention can be used for various purposes, and therefore, by blending the agent into a composition, a composition exhibiting a desired preventing or ameliorating effect can be obtained. For example, a composition including the GLP-1 secretion promotion agent of the present invention (hereinafter also referred to as a "composition of the present invention" or a "composition for promoting GLP-1 secretion") can be a composition for prevention or amelioration of diabetes, obesity, postprandial hyperglycemia, or neurodegenerative diseases.

When the GPR84 agonist is MCFA, the composition of the present invention is less likely to cause adverse reactions and can be preferably used as a composition suitable for continuous ingestion. In such a case, the ingestion period is not particularly limited, but can be set to, for example, 3 days or longer. The ingestion may be carried out every few hours, or may be carried out at intervals (for example, one day to several days).

The form of the composition of the present invention is not particularly limited, but it may be in any form of drugs, quasi-drugs, or foods.

### (Drugs, quasi-drugs)

The drug in the present invention may be pharmaceutical compositions for oral administration or pharmaceutical compositions for parenteral administration. Among these, pharmaceutical compositions for oral administration are preferable from the viewpoint of being easy to be ingested continuously.

Examples of forms of pharmaceutical compositions for oral administration include formulations such as capsules, tablets, pills, pulvis, fine granules, granules, liquids, and syrups. Pharmaceutical compositions for parenteral administration include formulations such as injections and infusions.

The drug of the present invention may include pharmacologically and pharmaceutically acceptable additives together with the GLP-1 secretion promotion agent of the present invention. As the "pharmacologically and pharmaceutically acceptable additive", a substance that is commonly used as an excipient or the like in the pharmaceutical field and that does not react with the active component included in the GLP-1 secretion promotion agent of the present invention can be used.

The dosage of the drug in the present invention can be appropriately set according to the purpose of administration (prevention or treatment), administration method, administration period, and other conditions (for example, symptoms, age, and body weight of a patient).

The aspect of the quasi-drugs in the present invention conforms to the above pharmaceuticals.

### (Foods)

Foods in the present invention include supplements, general foods, animal foods, and animal feeds.

The form of the supplement is not particularly limited, and may be a solid formulation or a liquid formulation. Examples thereof include formulations such as tablets, coated tablets, capsules, granules, pulvis, powders, sustained-release formulations, suspensions, emulsions, oral liquids, sugar-coated tablets, pills, fine granules, syrups, elixirs, and the like.

The form of general food is not particularly limited. Examples of the general foods include:
bakery products and confectionery (breads, cakes, cookies, biscuits, donuts, muffins, scones, chocolates, snacks, whipped cream, ice cream, ice cream mixes, confectionery bars,
chocolate bars, high fat bars, UHT desserts, pasteurized desserts, and the like),
beverages (fruit beverages, nutrition drinks, sports drinks, and the like),
seasonings and processed foods (dressings, sauces, mayonnaise, butter, margarine, prepared margarine, fat spreads, shortening, bakery mixes, and the like),
dairy products (cheese spreads, processed cheeses, dairy desserts, flavored milks, fermented milk products, cheese, butter, condensed milk products, yogurts, creams, and the like),
soups, stir-frying oils, frying oils, fried foods, processed meat products, frozen foods, fried foods, noodles, retort foods, liquid foods, swallowing foods, soybean products, pasteurized liquid eggs, gels, jelly, fillings (fat-based or hydrated filling),
and the like.

When the composition of the present invention is used for production of general food, the GPR84 agonist is preferably in the form of MCT. In such a case, it is preferable to add MCT to the raw material or replace the oil and fat of the raw material with MCT.

The amount of food ingestion in the present invention can be appropriately set according to the purpose of ingestion (prevention or treatment), the ingestion period, and other conditions (for example, symptoms, age, body weight of an ingesting person).

### <Composition for oral ingestion>

The GLP-1 secretion promotion agent of the present invention is preferably administered orally. Therefore, the GLP-1 secretion promotion agent of the present invention can be preferably prepared as a composition for oral ingestion.

The configuration and use of the composition for oral ingestion of the present invention may be the same as the above <Composition including GLP-1 secretion promotion agent of the present inventions

The composition for oral ingestion of the present invention is preferably administered so that 1 g or more of medium chain fatty acid is ingested per day. By administering 1 g or more per day, the effect of promoting GLP-1 secretion is obtained more easily.

The composition for oral ingestion of the present invention is preferably administered in a fasted state. By administering the composition in a fasted state, the effect of suppressing the increase in postprandial blood glucose level is obtained more easily.

The composition for oral ingestion of the present invention is preferably in the form of a beverage, seasoning, or other food (for example, in the same form as <Composition including GLP-1 secretion promotion agent of the present invention>) .

### <Screening method for GLP-1 secretion regulation agent>

As described above, the present invention is based on the novel finding that the secretion of GLP-1 is promoted through the binding of GPR84 agonists to GPR84. Therefore, the present invention also includes a screening method for determining whether a test substance can be a GLP-1 secretion regulation agent based on its binding property to GPR84.

Specifically, the screening method of the present invention includes:
a first measurement step of bringing a test substance into contact with GPR84-expressing cells, and measuring an amount of GLP-1 secretion from the GPR84-expressing cells;
a second measurement step of measuring an amount of GLP-1 secretion from the GPR84-expressing cells under the same conditions as in the first measurement step except that the test substance is not brought into contact with GPR84-expressing cells; and
a comparison step of comparing a measurement result of the first measurement step with a measurement result of the second measurement step. Then, in the comparison step, when a measured value of the first measurement step is higher or lower than a measured value of the second measurement step, the test substance is identified as a GLP-1 secretion regulation agent.

### (First measurement step)

In the first measurement step, the test substance is brought into contact with GPR84-expressing cells, and the amount of GLP-1 secretion from the GPR84-expressing cells is measured.

In the present invention, as the "test substance", any substance can be used. Examples of the test substance include proteins, peptides, fatty acids, and the like. The test substance may also include chemically synthesized low-molecular-weight compounds, and the like.

The "GPR84-expressing cells" of the present invention are not particularly limited as long as they are cells expressing GPR84 on any parts of a cell (cell surface, and the like). Examples of the GPR84-expressing cells include enteroendocrine cell lines (STC-1 cells, GLUTag cells) and the like. The origin of the GPR84-expressing cells is not particularly limited, but examples thereof include mammals (human, mouse, rat, rabbit, and the like).

The GPR84-expressing cells used in the screening method of the present invention may be isolated cells, isolated tissue (brain, bone marrow, gastrointestinal tract, and the like) including the cells, or a living body (excluding human) having the cells.

When the GPR84-expressing cells used in the screening method of the present invention are isolated cells, preferable examples are an isolated enteroendocrine cell line (STC-1 cells, GLUTag cells). As the isolated enteroendocrine cell line, a human-derived cell line is preferably used.

When the GPR84-expressing cells used in the screening method of the present invention are an isolated tissue, preferable examples include isolated tissue (brain, bone marrow, gastrointestinal tract, and the like). As the isolated tissue, human-derived tissue is preferably used.

When the GPR84-expressing cells used in the screening method of the present invention are living organisms, preferable examples are non-human mammals (mouse, rat, rabbit, and the like).

A method for bringing the test substance into contact with GPR84-expressing cells is not particularly limited. When isolated cells or tissues are used as the GPR84-expressing cells, for example, a method for culturing the GPR84-expressing cells in a cell culture medium in the presence of a test substance. Conventionally known conditions can be used for culture conditions and the like. When a living body is used as the GPR84-expressing cells, the method includes, for example, a method for administering (orally administering and the like) a test substance.

The method for measuring an amount of GLP-1 secretion from the GPR84-expressing cells is not particularly limited, and can be selected according to types of measurement targets. For example, conventionally known measuring methods with respect to hormones (ELISA and the like) can be employed. Measurement targets include post-culture media and biological samples (described later).

The measurement target in the first measurement step may be an amount of biologically active GLP-1.

In the first measurement step, the test substance is brought into contact with GPR84-expressing cells in a presence of medium chain fatty acid. As a result, it is possible to carry out screening of GLP-1 secretion regulation agents that also have a function of affecting the ligand activity of medium chain fatty acid in a state in which a medium-chain fatty acid is bound to GPR84 in the GPR84-expressing cells. A method of bringing the test substance into contact with the GPR84-expressing cells in the presence of medium chain fatty acid includes a method for culturing the GPR84-expressing cells in a culture medium supplemented with a medium-chain fatty acid in the presence of the test substance. For culture conditions, conventionally known conditions can be employed.

Examples of "substances that have a function of affecting the ligand activity of medium chain fatty acid" in the present invention include the following.
(1) A substance that binds to GPR84 at a site different from medium chain fatty acid to strengthen or weaken a signal sent by GPR84 to the cells.
(2) A substance that strengthens or weakens any signal in the intracellular signaling system (GLP-1 secretion pathway) generated by the binding of medium chain fatty acid with GPR 84.

### (Second measurement step)

In a second measurement step, GLP-1 secretion from GPR84-expressing cells is measured under the same conditions as in the first measurement step except that contact with the test substance is not carried out. Therefore, the cells used, culture conditions, measurement conditions, and the like, in the second measurement step are the same as in the first measurement step.

### (Comparison step)

In the comparison step, a measurement result of the first measurement step is compared with a measurement result of the second measurement step. In the comparison step, when a measured value in the first measurement step is higher or lower than the measured value in the second measurement step, the test substance is identified as a GLP-1 secretion regulation agent.

When the measured value in the first measurement step is higher than the measured value in the second measurement step, the test substance is identified as a GLP-1 secretion promotion agent. In the present invention, "the measured value in the first measurement step is higher than the measured value in the second measurement step" means that the measured value in the first measurement step is, for example, twice or more as high as the measured value in the second measurement step.

When the measured value in the first measurement step is lower than the measured value in the second measurement step, the test substance is identified as a GLP-1 secretion suppressing agent. In the present invention, "the measured value in the first measurement step is lower than the measured value in the second measurement step" means that the measured value in the first measurement step is, for example, 1/2 or less times lower than the measured value in the second measurement step.

When the measured value of the first measurement step is equivalent to the measured value of the second measurement step, the test substance is identified as an agent that does not affect GLP-1 secretion. In the present invention, "the measured value in the first measurement step is equivalent to the measured value in the second measurement step" means that the measured value in the first measurement step is, for example, more than 1/2 times or less than 2 times than the measured value in the second measurement step.

The present invention includes also a GLP-1 secretion regulation agent identified by the screening method mentioned above. The present invention also includes a composition for prevention or amelioration of diabetes, obesity, postprandial hyperglycemia, neurodegenerative diseases, hypoglycemia, adiposity, or nesidioblastosis, and including the GLP-1 secretion regulation agent identified by the screening method mentioned above.

### <Method for promoting secretion of GLP-1>

As described above, the present invention is based on the novel finding that the secretion of GLP-1 is promoted through the binding of GPR84 agonists to GPR84. Therefore, the present invention also includes a method for promoting secretion of GLP-1 in GPR84-expressing cells, the method including bringing GPR84-expressing cells into contact with a GPR84 agonist (a medium chain fatty acid and the like).

In the present invention, "promoting of GLP-1 secretion in GPR84-expressing cells" means that an amount of GLP-1 secretion from the GPR84-expressing cells is increased compared with the case without contact with a GPR84 agonist. Such an increasing effect is mainly based on activation of the GLP-1 secretory pathway in GPR84-expressing cells.

### <Production method of composition for promoting GLP-1 secretion>

As described above, the present invention is based on the novel finding that the secretion of GLP-1 is promoted through the binding of GPR84 agonists to GPR84. Therefore, the present invention also includes a production method of a composition for promoting secretion of GLP-1 using a GPR84 agonist identified based on the binding to GPR84.

Specifically, the production method of a composition for promoting GLP-1 secretion of the present invention includes the following two aspects.

### <Production method-1>

A first aspect of a production method of a composition for promoting secretion of GLP-1 of the present invention is as follows. A production method of a composition for promoting secretion of GLP-1, the method including:
a contact step of bringing a GPR84 agonist candidate substance into contact with GPR84-expressing cells in vitro;
a measurement step of measuring an amount of GLP-1 secretion from the GPR84-expressing cells before and after the contact step; and
an identification step of identifying a GPR84 agonist, in which an amount of GLP-1 secretion after the contact step is increased to more than an amount of GLP-1 secretion before the contact step, as a GLP-1 secretion promotion agent.

### (Contact step)

In the contact step, the GPR84 agonist candidate substance and GPR84-expressing cells are brought into contact with each other in vitro.

As the "GPR84 agonist candidate substance" in the present invention, any substance can be used. Examples of the GPR84 agonist candidate substance include proteins, peptides, fatty acids, and the like.

The GPR84 agonist candidate substance may be an agonist of human GPR84. Examples of the human GPR84 agonist include medium chain fatty acid, protein, peptide, fatty acids other than medium chain fatty acids, and the like.

The GPR84 agonist candidate substance may be an orally administrable substance. Examples of such substances include medium chain fatty acids, proteins, peptides, and fatty acids other than medium chain fatty acids, and the like.

A GPR84 agonist candidate substance may be a selective GPR84 agonist. Examples of the selective GPR84 agonists include proteins, peptides, fatty acids, and the like.

The selective GPR84 agonist candidate substance may have EC₅₀ of less than 10 µM.

The GPR84 agonist candidate substance may be a low-molecular-weight compound. In the present invention, the "low-molecular-weight compound" refers to any compounds having a molecular weight of 200 or less.

The GPR84 agonist candidate substance may be medium chain fatty acid. The medium chain fatty acid may be the components (examples of the GPR84 agonist) mentioned above.

A method for bringing the GPR84 agonist candidate substance into contact with the GPR84-expressing cells is not particularly limited, and examples of the method include a method of culturing GPR84-expressing cells in a cell culture medium in the presence of the GPR84 agonist candidate substance. For culture conditions and the like, conventionally known conditions can be employed.

### (Measurement step)

In the measurement step, the amount of GLP-1 secreted from GPR84-expressing cells is measured before and after the contact step. In other words, the GLP-1 concentration in the culture medium of GPR84-expressing cells before the contact step and the GLP-1 concentration in the culture medium of GPR84-expressing cells after the contact step are measured. The amount of GLP-1 secretion from the GPR84-expressing cells before the contact step is preferably measured on the culture medium collected within 30 minutes before the contact step. The amount of GLP-1 secretion from the GPR84-expressing cells after the contact step is preferably measured on culture medium collected within 60 minutes after the contact step. When the collection time of the culture medium before and after the contact step is within the above range, the repeatability of measurement is further improved.

The method for measuring the amount of GLP-1 secreted from GPR84-expressing cells is not particularly limited, but the method can be selected according to the type of measurement targets. For example, conventionally known measuring methods with respect to hormones (ELISA, and the like) can be employed.

The measurement target in the measurement step may be an amount of biologically active GLP-1.

### (Identification step)

In the identification step, a GPR84 agonist, in which an amount of GLP-1 secretion after the contact step is increased to more than an amount of GLP-1 secretion before the contact step and after the measurement step, is identified as a GLP-1 secretion promotion agent. In the present invention, "the amount of GLP-1 secretion after the contact step is increased to more than an amount of GLP-1 secretion before the contact step" means that the measured value after the contact step is, for example, twice or more as high as the measured value before the contact step.

<Production method-2>

A second aspect of the production method of a composition for promoting secretion of GLP-1 of the present invention is as follows. A production method of a composition for promoting secretion of GLP-1, the method including:
an administration step of administering a GPR84 agonist candidate substance to a mammal (excluding humans);
a sample obtaining step of obtaining a biological sample from the mammal before and after the administration step;
a measurement step of measuring an amount of GLP-1 in the biological sample; and
an identification step of identifying a GPR84 agonist candidate substance before and after the measurement step, in which an amount of GLP-1 secretion after the administration step is increased to more than an amount of GLP-1 secretion before the administration step and after the measurement step, as a GLP-1 secretion promotion agent.

Hereinafter, matters common to the <Production method-1> will be appropriately omitted.

### (Administration step)

In the administration step, a GPR84 agonist candidate substance is administered to a mammal.

Mammals include, but are not limited to, mice, rats, rabbits, and the like. However, humans are excluded.

The dosage of the GPR84 agonist candidate substance in the administration step is not particularly limited, but may be 500 to 2000 mg/kg body weight.

The administration method of the GPR84 agonist candidate substance in the administration step is not particularly limited, but the administration method, may be, for example, oral administration.

### (Sample obtaining step)

In the sample obtaining step, a biological sample is obtained from a mammal before and after the administration step. In other words, the same type of biological sample is obtained from each of a mammal before being subjected to the administration step and the mammal after being subjected to the administration step.

In the present invention, the "biological sample" refers to any substance that can be isolated from living organisms. Specific biological samples include, but are not limited to, blood (plasma, serum) and the like. Of these, blood is preferable because of ease of sample handling.

The biological sample before the administration step is preferably obtained within 60 minutes before the administration step. A biological sample after the administration step is preferably obtained within 120 minutes after the administration step. When the obtaining time of a biological sample before and after the administration step is within the above range, repeatability of measurement is further improved.

### (Measurement step)

In the measurement step, amounts of GLP-1 in the biological sample obtained before and after the administration step are measured. The method for measuring amounts of GLP-1 is not particularly limited, but can be selected according to the type of biological sample. For example, conventionally known measuring methods with respect to hormones (ELISA, and the like) can be employed.

A measurement target in the measurement step may be an amount of biologically active GLP-1.

### (Identification step)

In the identification step, a GPR84 agonist candidate substance, in which an amount of GLP-1 secretion after the administration step is increased to more than an amount of GLP-1 secretion before the administration step and after the measurement step is identified as a GLP-1 secretion promotion agent. In the present invention, "the amount of GLP-1 secretion after the administration step is increased to more than the amount of GLP-1 secretion before the administration step" means that the measured value after the administration step is, for example, twice or more as high as the measured value before the administration step.

### <Additional steps in production methods 1 and 2>

After the identification step, the above production methods 1 and 2 may further include any one or more of the following steps. The order of the steps below is not particularly limited.

### (Composition preparation step)

In the composition preparation step, a composition including the GLP-1 secretion promotion agent identified in the identification step and a pharmaceutically acceptable carrier is prepared. Through this step, a distributable composition for promoting GLP-1 secretion can be prepared.

As for the form and components of the composition, those mentioned in the above <Composition including GLP-1 secretion promotion agent of the present invention> can be employed.

### (Structural analysis step)

In the structural analysis step, the structure of the GPR84 agonist candidate substance is analyzed after the identification step. This step is useful when the structure of the GPR84 agonist candidate substance is unknown.

The method of structural analysis is not particularly limited, but examples of the method include X-ray analysis, mass spectrometry, and the like.

### (Synthesis step)

In the synthesis step, after the identification step, a GPR84 agonist candidate substance is synthesized. Through this step, a desired amount of GPR84 agonist, which is an active component of a GLP-1 secretion promotion agent, can be prepared.

The synthesis method is not particularly limited, but examples of the method include microbial culture and the like.

Note here that when the structure of the GPR84 agonist candidate substance is unknown, it is preferable to carry out the structural analysis step before the synthesis step.

### <Use of GPR84 agonist>

As described above, the present invention is based on the novel finding that the secretion of GLP-1 is promoted through the binding of GPR84 agonists to GPR84. Promotion of GLP-1 secretion provides therapeutic benefits for various diseases. Therefore, the present invention includes use of a GPR84 agonist for producing therapeutic agent for disease, wherein the disease is one or more diseases selected from the group consisting of diabetes, obesity, postprandial hyperglycemia, and neurodegenerative diseases.

### EXAMPLES

Hereinafter, the present invention will be described in more detail with reference to Examples, but the present invention is not limited to these Examples.

### <Test 1> Examination-1 of effect of medium chain fatty acid on GLP-1 secretion

By carrying out a cell culture test (in vitro test) by the following method, and administering various medium chain fatty acids (capric acid or lauric acid, both are a free form) to enteroendocrine cells (STC-1 cells), an effect of each medium chain fatty acid on GLP-1 secretion was examined. The above medium chain fatty acids correspond to GPR84 agonists.

### (1) Preparation of samples

Various medium chain fatty acid samples were prepared by the following method. A dimethylsulfoxide sample was also prepared as a control.

### (1-1) Control Sample

Dimethyl sulfoxide (trade name "Dimethyl Sulfoxide", manufactured by FUJIFILM Wako Pure Chemical Industries, Ltd.) was used as a control sample.

### (1-2) Capric acid sample

To the same dimethyl sulfoxide (1 ml) as in the (1-1) above, 172 mg of capric acid (trade name "Decanoic Acid <Capric Acid>", manufactured by New Check Prep) was added to obtain 1000 mM of capric acid samples. A 1000 mM capric acid sample (0.1 ml) was added to dimethylsulfoxide (0.9 ml) to obtain a 100 mM capric acid sample. A 100 mM capric acid sample (0.1 ml) was added to dimethylsulfoxide (0.9 ml) to obtain a 10 mM capric acid sample. Note here that when capric acid was added to dimethylsulfoxide, a mixture was mixed while heating to prepare a sample having a uniform capric acid concentration.

### (1-3) Lauric acid sample

To the same dimethylsulfoxide (1 ml) as in the (1-1) above, 200 mg of lauric acid (trade name "Dodecanoic Acid <Lauric Acid>", manufactured by New Check Prep) was added to obtain 1000 mM of lauric acid sample. A 1000 mM lauric acid sample (0.1 ml) was added to dimethylsulfoxide (0.9 ml) to obtain a 100 mM lauric acid sample. A 100 mM lauric acid sample (0.1 ml) was added to dimethylsulfoxide (0.9 ml) to obtain a 10 mM lauric acid sample. Note here that when lauric acid was added to dimethyl sulfoxide, the mixture was mixed while heating to prepare a sample having a uniform lauric acid concentration.

### (2) Preparation of STC-1 Cells

STC-1 cells (trade name "STC-1", manufactured by American Type Culture Collection) were cultured in a medium at 37°C in 5% CO₂ for 48 hours to obtain STC-1 cells for testing. Note here that as the medium, a DMEM medium (trade name "Dulbecco's Modified Eagle Medium (High Glucose)", manufactured by Sigma-Aldrich) including 15% horse serum (trade name "Horse Serum, New Zealand origin", manufactured by Gibco) and 5% fetal bovine serum (trade name "Fetal Bovine Serum (FBS) Standard, Origin South America", manufactured by Cosmo Bio) was used.

### (3) Cell Culture Test

The culture medium for STC-1 cells was replaced with a serum-free medium (300 ul/well), starvation was carried out for 30 minutes, then the serum-free medium was replaced with a new serum-free medium (300 µl/well), and 0.3 µl each of any one of samples was added to each well. Thirty minutes after administration of the sample, the medium was collected and mixed in a 1.5 ml tube in which 3 µL of DPP4 inhibitor (trade name "DPP IV Inhibitor", manufactured by Sigma-Aldrich) had been placed in advance. Then, each tube was centrifuged at 4°C at 1,000 × g for 5 minutes, and the supernatant was collected in a new 1.5 ml tube.

### (4) Measurement of GLP-1 concentration

GLP-1 concentration (nM) in the collected supernatant was measured. Note here that the GLP-1 concentration was measured by ELISA method using a commercially available kit (trade name "Glucagon Like Peptide-1 (Active) ELISA", manufactured by Sigma-Aldrich).

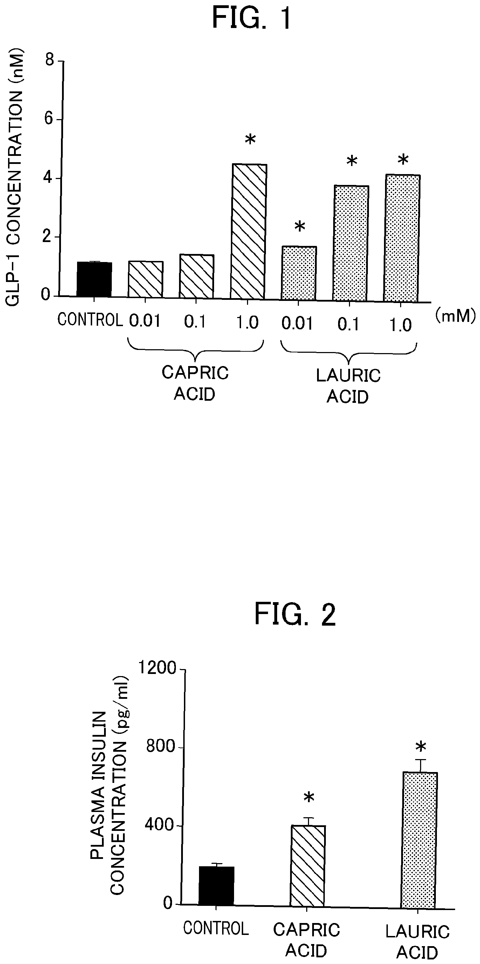

The results of the cell culture test are shown in FIG. 1 and Table 1. In the data of FIG. 1 and Table 1, the measured values of GLP-1 concentration are expressed as "mean ± standard error". Dunnet's test was used to test significant differences between trials, and significant differences were determined to exist when the risk rate was less than 5% (* p < 0.05). Also, the sample concentrations in FIG. 1 and Table 1 indicate the concentrations in the medium in the cell culture test.

As shown in Figure 1 and Table 1, both capric acid and lauric acid promoted GLP-1 secretion from STC-1 cells. Such a promotion effect tended to become more remarkable as the concentration of each medium chain fatty acid became higher. In addition, lauric acid tended to have a higher effect of promoting GLP-1 secretion than capric acid.

**[Table 1]**

| Table 1: Results of cell culture test | | |
|---|---|---|
| | | GLP-1 CONCENTRATION (nM) |
| Control | | 1.156±0.030 |
| Capric acid | 0.01 mM | 1.235±0.120 |
| | 0.1 mM | 1.517±0.005 |
| | 1.0 mM | 4.629±0.087 * |
| Lauric acid | 0.01 mM | 1.827±0.060 * |
| | 0.1 mM | 3.915±0.197 * |
| | 1.0 mM | 4.268±0.084 * |

### <Test 2> Examination of effect of medium chain fatty acid on insulin secretion

A glucose tolerance test was carried out by the following method to examine the effect by administration of various medium chain fatty acids (capric acid or lauric acid, both are a free body) to mice on plasma insulin secretion at that time. Note here that the above-mentioned medium chain fatty acid corresponds to a GPR84 agonist.

### (1) Preparation of samples

Various medium chain fatty acid samples and glucose solutions for glucose tolerance were prepared by the following method. Note here that a carboxymethylcellulose sample was also prepared as a control.

### (1-1) Control Sample

Ten tablets of PBS (trade name "Phosphate Buffered Salts Tablets", manufactured by Takara Bio Inc.) were dissolved in distilled water, and the total amount was adjusted to 1000 ml. To 10 ml of the prepared solution, 50 mg of carboxymethyl cellulose (trade name "Carboxymethyl cellulose sodium", manufactured by FUJIFILM Wako Pure Chemical Industries, Ltd.) was added and mixed to obtain a control sample. Note here that carboxymethylcellulose is a component known as a carrier for pharmaceuticals and the like.

### (1-2) Capric acid Sample

Ten tablets of PBS (trade name "Phosphate Buffered Salts Tablets", manufactured by Takara Bio Inc.) were dissolved in distilled water, and the total volume was adjusted to 1000 ml. To 10 ml of the prepared solution, 50 mg of carboxymethyl cellulose (trade name "Carboxymethyl cellulose sodium", manufactured by FUJIFILM Wako Pure Chemical Industries, Ltd.) was added and mixed to obtain a carrier solution. To the obtained carrier solution, 2 g of capric acid (trade name "Decanoic acid", manufactured by FUJIFILM Wako Pure Chemical Industries, Ltd.) was added, and mixed while heating to obtain a capric acid sample.

### (1-3) Lauric acid sample

A carrier solution was obtained in the same manner as in the (1-2) above. To the obtained carrier solution, 2 g of lauric acid (trade name "Dodecanoic acid", manufactured by Sigma-Aldrich) was added and mixed while heating to obtain a lauric acid sample.

### (1-4) Glucose solution

Ten tablets of PBS (trade name "Phosphate Buffered Salts Tablets", manufactured by Takara Bio Inc.) were dissolved in distilled water, and the total amount was adjusted to 1000 ml. To 10 ml of the prepared solution, 2.5 g of glucose (trade name "D(+)-glucose", manufactured by FUJIFILM Wako Pure Chemical Industries, Ltd.) was added to obtain a glucose liquid.

### (2) Glucose tolerance test

Based on the following method, any one of a control sample, a capric acid sample, and a lauric acid sample was administered to mice in each group, and then a glucose solution was further administered to carry out a glucose tolerance test.

### (2-1) Preparation of mice

C57BL/6 male mice at 7- to 9-weeks old were purchased from Japan SLC, Inc. Each mouse (15 mice in total) was acclimatized for 1 week and then divided into three groups (a control sample administration group, a capric acid sample administration group, and a lauric acid sample administration group) so that the average body weight was uniform.

Note here that the mice were reared in plastic cages under an environment of temperature of 24±1°C, humidity of 50±10%, and a 12-hour light-dark cycle (lighting from 8:00 to 20:00). Water and solid feed (trade name "CLEA Rodent Diet CE-2 for mice, rats and hamsters", manufactured by CLEA Japan Co., Ltd.) were fed ad libitum. However, the day before the glucose tolerance test, fasting was started from 17:00, and on the day of the test, the administration was started from 10:00.

### (2-2) Administration of samples and glucose tolerance

A glucose solution was administered to each group of mice, 60 minutes after administration of each sample (any one of a control sample, a capric acid sample, or a lauric acid sample). Note here that the mice were 8 to 10 weeks old at the time of administration.

Each of the samples (a control sample, a capric acid sample, a lauric acid sample, and a glucose solution) was orally administered to mice using a sonde.

The dosage of each sample was set as follows.
Control sample, capric acid sample, lauric acid sample: 1000 mg/kg body weight of each mouse
Glucose solution: 2.5 g/kg body weight of each mouse

### (3) Measurement of plasma insulin concentration

For each group, blood was collected from the inferior vena cava 30 minutes after administration of the glucose solution. Plasma was prepared from the obtained blood by a conventional method, and the plasma insulin concentration (pg/ml) was measured. The plasma insulin concentration was measured by ELISA using a commercially available kit (trade name "Revis (trademark) Insulin-Mouse (RTU)", manufactured by Fujifilm Wako Shibayagi Co., Ltd.).

The test results are shown in FIG. 2 and Table 2. In the data of FIG. 2 and Table 2, the measured value of the plasma insulin concentration was expressed as "mean ± standard error". Dunnet's test was used to test significant differences between trials, and significant differences were determined to exist when the risk rate was less than 5% (*p<0.05).

**[Table 2]**

| Table 2: Measurement results of plasma insulin concentration | |
|---|---|
| | Plasma insulin concentration (pg/ml) |
| Control | 207.6±8.2 |
| Capric acid | 395.1±26.1 * |
| Lauric acid | 702.8±84.4 * |

As shown in FIG. 2 and Table 2, both capric acid and lauric acid significantly increased plasma insulin concentrations. Furthermore, lauric acid tended to have a higher effect of increasing the plasma insulin concentration than capric acid.

### <Test 3> Examination-2 of effect of medium chain fatty acid on GLP-1 secretion

An administration test was carried out by the following method to examine an effect of administration of various medium chain fatty acids (capric acid or lauric acid, both are a free form) to mice on plasma GLP-1 secretion. The above medium chain fatty acids correspond to GPR84 agonists.

### (1) Sample Preparation

A control sample (carboxymethylcellulose), a capric acid sample, and a lauric acid sample were prepared in the same manner as in the <Test 2> above.

### (2) Administration test

Based on the following method, any one of a control sample, a capric acid sample, and a lauric acid sample was administered to mice in each group.

### (2-1) Preparation of mice

In this test, two types of mice, that is, wild-type mice and GPR84 knockout mice, were prepared. As wild-type mice, 18 C57BL/6 male mice at 7- to 9-weeks old (purchased from Japan SLC, Inc.) were used. As the GPR84 knockout mice, 12 mice were prepared using C57BL/6 according to the method by Wang et al. (Pharmacological Research Volume 164, February 2021, 105406). Furthermore, the age in weeks of the GPR84 knockout mice was the same as that of the wild-type mice. Each mouse was acclimatized for one week, and then divided into six groups (a control sample-administered group, a capric acid sample-administered group, and a lauric acid sample administration group for each of the wild-type mice and the GPR84 knockout mice) so that average value of the body weight is uniform in each group.

Note here that the mice were reared in plastic cages under an environment of temperature of 24±1°C, humidity of 50±10%, and a 12-hour light-dark cycle (lighting from 8:00 to 20:00). Water and solid feed (trade name "CLEA Rodent Diet CE-2 for mice, rats and hamsters", manufactured by CLEA Japan Co., Ltd.) were fed ad libitum. However, the day before the glucose tolerance test, fasting was started from 17:00, and on the day of the test, the administration was started from 10:00.

### (2-2) Administration of sample

Each sample (any one of a control sample, a capric acid sample, and a lauric acid sample) was administered to each group of mice after blood was collected from the tail vein. Note here that the mice were 8 to 10 weeks old at the time of administration.

Each of the samples (a control sample, a capric acid sample, and a lauric acid sample) was orally administered to mice using a sonde. The dosage of each sample was set at 1000 mg per kg body weight of mice.

### (3) Measurement of plasma GLP-1 concentration

Blood was collected from the tail vein 120 minutes after administration of each sample. Plasma was prepared by a conventional method from the blood obtained before and after sample administration, and the plasma GLP-1 concentration (pM) was measured. The plasma GLP-1 concentration was measured by ELISA using a commercially available kit (trade name "Glucagon Like Peptide-1 (Active) ELIS", manufactured by Sigma-Aldrich).

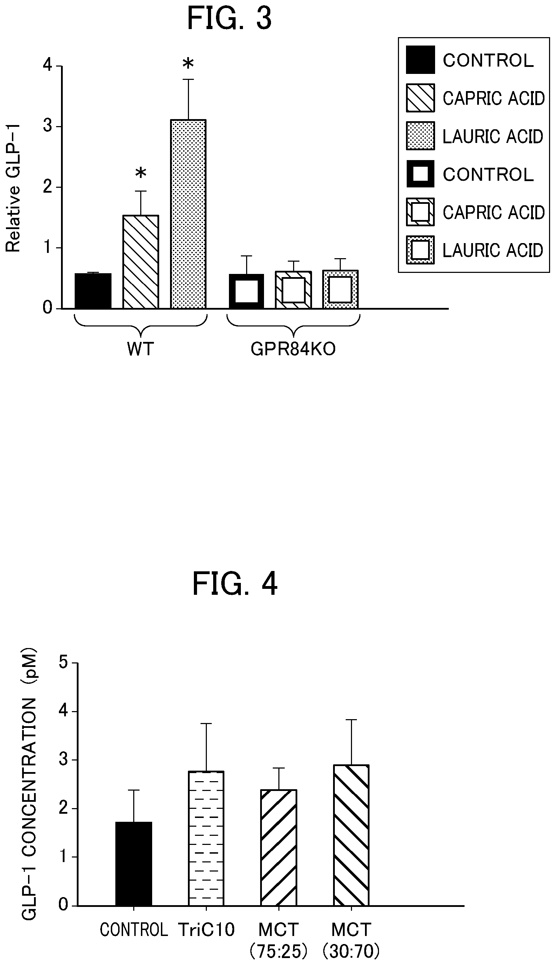

The test results are shown in FIG. 3 and Table 3. The data in FIG. 3 and Table 3 are expressed as relative values (Relative GLP-1) when the average value of plasma GLP-1 concentration before administration of each sample is set to "1", and expressed as "mean ± standard error of the relative values". Dunnet's test was used to test significant differences between trials, and significant differences were determined to exist when the risk rate was less than 5% (*p<0.05).

In FIG. 3 and Table 3, "WT" refers to wild-type mice, and "GPR84KO" refers to GPR84 knockout mice.

**[Table 3]**

| Table 3: Measurement of plasma GLP-1 concentration | | |
|---|---|---|
| | | Relative GLP-1 |
| WT | Control | 0.21±0.03 |
| | Capric acid | 1.65±0.29 * |
| | Lauric acid | 3.02±0.67 * |
| GPR84KO | Control | 0.62±0.27 |
| | Capric acid | 0.63±0.2 |
| | Lauric acid | 0.68±0.19 |

As shown in "WT" in FIG. 3 and Table 3, both capric acid and lauric acid significantly increased the plasma GLP-1 concentration. Furthermore, lauric acid tended to have a higher effect of increasing the plasma GLP-1 concentration than capric acid.

On the other hand, as shown in "GPR84KO" in FIG. 3 and Table 3, in GPR84 knockout mice, regardless of the type of medium chain fatty acid or the presence or absence of medium chain fatty acid administration, no change in plasma GLP-1 concentration was observed. This shows that the effect of increasing the plasma GLP-1 concentration by capric acid and lauric acid is brought via GPR84. Furthermore, the increase in the plasma insulin concentration observed in the <Test 2> above was presumed to be due to an increase in the plasma GLP-1 concentration brought via GPR84.

Although the data are not shown, when the same test as the <Test 2> above was carried out using GPR84 knockout mice, both capric acid and lauric acid increased the plasma insulin concentration. However, the increasing effect was lower than with wild-type mice. Based on this result and the results of the <Test 3> above, in GPR84 knockout mice, among insulin secretion signaling pathways (a plurality of pathways is known to be present in vivo), while pathways in which the GLP-1 concentration increases were blocked, other pathways functioned normally. It was inferred that the total insulin secretion was reduced compared with that of wild-type mice.

### <Test 4> Examination of effect of triacylglycerols on GLP-1 secretion

A glucose tolerance test was carried out by the following method to examine effects of administration of triacylglycerol to mice on the plasma GLP-1 secretion during glucose tolerance.

### (1) Preparation of samples

Various triacylglycerol samples and glucose solutions for glucose tolerance were prepared by the following methods. A PBS solution was also prepared as a control.

### (1-1) Control sample

Ten tablets of PBS (trade name "Phosphate Buffered Salts Tablets", manufactured by Takara Bio Inc.) were dissolved in distilled water, and the total amount was adjusted to 1000 ml to obtain a PBS solution (control sample).

### (1-2) TriC10 sample

To the PBS solution (10 ml) obtained in the same manner as in the (1-1) above, 50 mg of carboxymethyl cellulose (trade name "Carboxymethyl cellulose sodium", manufactured by FUJIFILM Wako Pure Chemical Industries, Ltd.) was added and mixed to obtain a carrier solution. To the obtained carrier solution, 2 g of triacylglycerol (trade name "Tridecanoin <Tricaprin>", manufactured by New Check Prep Co., Ltd.) in which the constituent fatty acids were all capric acid was added and mixed while heating to obtain TriC10 sample.

### (1-3) MCT (75:25) sample

A carrier solution was obtained in the same manner as in the (1-2) above. To the obtained carrier solution, 2 g of medium chain fatty acid triacylglycerol (trade name "O.D.O", manufactured by The Nisshin OilliO Group, Ltd.) in which the constituent fatty acid is only caprylic acid and capric acid, and the mass ratio of caprylic acid : capric acid was 75 : 25, was added and mixed to obtain an MCT (75:25) sample.

### (1-4) MCT (30:70) sample

A carrier solution was obtained in the same manner as in the (1-2) above. To the obtained carrier solution, 2 g of medium chain fatty acid triacylglycerol (trade name "Nisshin MCT C10R", manufactured by The Nisshin OilliO Group, Ltd.) in which the constituent fatty acid is only caprylic acid and capric acid, and the mass ratio of caprylic acid : capric acid was 30 : 70, was added and mixed to obtain an MCT (30 : 70) sample.

### (1-5) Glucose solution

Ten tablets of PBS (trade name "Phosphate Buffered Salts Tablets", manufactured by Takara Bio Inc.) were dissolved in distilled water, and the total amount was adjusted to 1000 ml. To 10 ml of the prepared solution, 2.5 g of glucose (trade name "D(+)-glucose", manufactured by FUJIFILM Wako Pure Chemical Industries, Ltd.) was added to obtain a glucose solution.

### (2) Glucose tolerance test

Based on the following method, a glucose tolerance test was carried out by administering any one of a control sample, a TriC10 sample, an MCT (75:25) sample, and an MCT (30:70) sample, and then further administrating a glucose solution to mice in each group.

### (2-1) Preparation of mice

C57BL/6 male mice at 7- to 9-weeks old were purchased from Japan SLC, Inc. Each mouse (12 mice in total) was acclimated for one week, and then divided into four groups (a control sample administration group, a TriC10 sample administration group, an MCT (75:25) sample administration group, and an MCT (30:70) sample administration group) so that average value of the body weight is uniform in each group.

Note here that the mice were reared in plastic cages under an environment of temperature of 24±1°C, humidity of 50±10%, and a 12-hour light-dark cycle (lighting from 8:00 to 20:00). Water and solid feed (trade name "CLEA Rodent Diet CE-2 for mice, rats and hamsters", manufactured by CLEA Japan Co., Ltd.) were fed ad libitum. However, the day before the glucose tolerance test, fasting was started from 17:00, and on the day of the test, the administration was started from 10:00.

### (2-2) Administration of samples and glucose tolerance

A glucose solution was administered to each group of mice, 60 minutes after administration of each sample (any one of a control sample, a TriC10 sample, an MCT (75:25) sample, and an MCT (30:70) sample). Note here that the mice were 8 to 10 weeks old at the time of administration.

Each sample (a control sample, a TriC10 sample, an MCT (75:25) sample, an MCT (30:70) sample, and a glucose solution) was orally administered to mice using a sonde.

The dosage of each sample was set as follows.
Control sample, TriC10 sample, MCT (75:25) sample, MCT (30:70) sample: 300 ul for each mouse
Glucose solution: 2.5 g/kg body weight of each mouse

### (3) Measurement of plasma GLP-1 concentration

For each group, blood was collected from the inferior vena cava 30 minutes after administration of the glucose solution. Plasma was prepared from the obtained blood by a conventional method, and the plasma GLP-1 concentration (pM) was measured. Note here that the plasma GLP-1 concentration was measured by ELISA using a commercially available kit (trade name "Glucagon Like Peptide-1 (Active) ELISA", manufactured by Sigma-Aldrich).

The test results are shown in FIG. 4 and Table 4. Note here that in the data of FIG. 4 and Table 4, the measured value of plasma GLP-1 concentration was expressed as "mean ± standard error".

As shown in FIG. 4 and Table 4, both triacylglycerols increased the plasma GLP-1 concentrations. These results show that the advantageous effect of the present invention is exhibited even when medium chain fatty acid is administered in the form of triacylglycerol instead of the free form.

**[Table 4]**

| Table 4: Measurement of plasma GLP-1 concentration | |
|---|---|
| | GLP-1 CONCENTRATION (pM) |
| Control | 1.742±0.687 |
| TriC10 | 2.803±0.952 |
| MCT (75:25) | 2.409±0.453 |
| MCT (30:70) | 2.929±0.935 |

## Claims

1. A screening method for a GLP-1 secretion regulation agent, the method including:
a first measurement step of bringing a test substance into contact with GPR84-expressing cells, and measuring an amount of GLP-1 secretion from the GPR84-expressing cells;
a second measurement step of measuring an amount of GLP-1 secretion from the GPR84-expressing cells under same conditions as in the first measurement step except that the test substance is not brought into contact with GPR84-expressing cells; and
a comparison step of comparing a measurement result of the first measurement step with a measurement result of the second measurement step,
wherein, in the comparison step, when a measured value of the first measurement step is higher or lower than a measured value of the second measurement step, the test substance is identified as a GLP-1 secretion regulation agent.

2. The screening method according to claim 1, wherein, in the first measurement step, the test substance is brought into contact with the GPR84-expressing cells in a presence of medium chain fatty acid.

3. A GLP-1 secretion regulation agent, being identified by the screening method according to claim 1 or claim 2.

4. A composition for prevention or amelioration of diabetes, obesity, postprandial hyperglycemia, neurodegenerative diseases, hypoglycemia, adiposity, or nesidioblastosis, the composition comprising the GLP-1 secretion regulation agent according to claim 3.

5. A method for promoting secretion of GLP-1 in GPR84-expressing cells, the method comprising bringing a GPR84 agonist into contact with GPR84-expressing cells.

6. A production method of a composition for promoting secretion of GLP-1, the method comprising:
a contact step of bringing a GPR84 agonist candidate substance into contact with GPR84-expressing cells in vitro;
a measurement step of measuring an amount of GLP-1 secretion from the GPR84-expressing cells before and after the contact step; and
an identification step of identifying a GPR84 agonist candidate substance, in which an amount of GLP-1 secretion after the contact step is increased to more than an amount of GLP-1 secretion before the contact step and after the measurement step, as a GLP-1 secretion promotion agent.

7. A production method of a composition for promoting secretion of GLP-1, the method comprising:
an administration step of administering a GPR84 agonist candidate substance to a mammal excluding a human;
a sample obtaining step of obtaining a biological sample from the mammal before and after the administration step;
a measurement step of measuring an amount of GLP-1 in the biological sample obtained before and after the administration step; and
an identification step of identifying a GPR84 agonist candidate substance, in which an amount of GLP-1 secretion after the administration step is increased to more than an amount of GLP-1 secretion before the administration step, as a GLP-1 secretion promotion agent.

8. The production method according to claim 7, wherein the biological sample is blood.

9. The production method according to any one of claim 6 to claim 8, further comprising a composition preparation step of preparing a composition including the GLP-1 secretion promotion agent identified in the identification step and a pharmaceutically acceptable carrier.

10. The production method according to any one of claim 6 to claim 9, further comprising a structure analysis step of analyzing a structure of the GPR84 agonist candidate substance after the identification step.

11. The production method according to any one of claim 6 to claim 10, further comprising a synthesis step of synthesizing the GPR84 agonist candidate substance after the identification step.

12. The production method according to any one of claim 6 to claim 11, wherein the amount of GLP-1 secretion is an amount of biologically active GLP-1.

13. The production method according to any one of claim 6 to claim 12, wherein the GPR84 agonist candidate substance is a human GPR84 agonist.

14. The production method according to any one of claim 6 to claim 13, wherein the GPR84 agonist candidate substance is an orally administrable substance.

15. The production method according to any one of claim 6 to claim 14, wherein the GPR84 agonist candidate substance is a selective GPR84 agonist.

16. The production method according claim 15, wherein the selective GPR84 agonist candidate substance has EC₅₀ of less than 10 µM.

17. The production method according to any one of claim 6 to claim 16, wherein the GPR84 agonist candidate substance is a low-molecular-weight compound.

18. The production method according to any one of claim 6 to claim 17, wherein the GPR84 agonist candidate substance is medium chain fatty acid.

19. Use of a GPR84 agonist for producing a therapeutic agent for disease,
wherein the disease is one or more diseases selected from the group consisting of diabetes, obesity, postprandial hyperglycemia, and neurodegenerative diseases.

20. A GLP-1 secretion promotion agent comprising a GPR84 agonist as an active component.

21. The GLP-1 secretion promotion agent according to claim 20, wherein the GPR84 agonist is medium chain fatty acid.

22. The GLP-1 secretion promotion agent according to claim 21, wherein a number of carbon atoms of the medium chain fatty acid is 9 to 12.

23. The GLP-1 secretion promotion agent according to claim 21 or 22, wherein the medium chain fatty acid is in a form of constituent fatty acid of triacylglycerol and/or free fatty acid.

24. The GLP-1 secretion promotion agent according to claim 21, wherein the medium chain fatty acid is in a form of triacylglycerol, and
the triacylglycerol is one or more triacylglycerols selected from the group consisting of triacylglycerol including only medium chain fatty acid having 10 carbon atoms as the constituent fatty acid, and triacylglycerol including medium chain fatty acid having 8 carbon atoms and medium chain fatty acid having 10 carbon atoms as the constituent fatty acid.

25. The GLP-1 secretion promotion agent according to claim 24, wherein the triacylglycerol is
triacylglycerol including medium chain fatty acid having 8 carbon atoms and medium chain fatty acid having 10 carbon atoms as the constituent fatty acid, and
a mass ratio of the medium chain fatty acid having 8 carbon atoms to the medium chain fatty acid having 10 carbon atoms satisfies: the medium chain fatty acid having 8 carbon atoms:the medium chain fatty acid having 10 carbon atoms = 30:70 to 75:25.

26. The GLP-1 secretion promotion agent according to any one of claim 20 to claim 25, further comprising an active component being other than the GPR84 agonist and including a GLP-1 secretion promotion effect.

27. The GLP-1 secretion promotion agent according to any one of claim 20 to claim 26, which are administered in a fasted state.

28. The GLP-1 secretion promotion agent according to any one of claim 20 to claim 27, which is to be used for one or more purposes selected from the group consisting of:
preventing or ameliorating diabetes, obesity, postprandial hyperglycemia, and neurodegenerative diseases;
ameliorating neuropathy associated with diabetes;
suppressing glucagon secretion;
suppressing gastric excretion and/or gastrointestinal secretions;
suppressing appetite and/or food ingestion; and
promoting proliferation of pancreatic β cells.

29. A composition for prevention or amelioration of diabetes, obesity, postprandial hyperglycemia, or neurodegenerative diseases, the composition comprising a GLP-1 secretion promotion agent according to any one of claim 20 to claim 28.

30. The composition for prevention or amelioration according to claim 29, being in a form of a drug, a quasi-drug, or food.

31. A composition for oral ingestion, comprising a GLP-1 secretion promotion agent according to any one of claim 20 to claim 28.

32. The composition for oral ingestion according to claim 31, being administered so that 1 g or more per day of medium chain fatty acid is ingested.

33. The composition for oral ingestion according to claim 31 or claim 32, being administered in a fasted state.

34. The composition for oral ingestion according to any one of claim 31 to claim 33, being in a form of a beverage, seasoning, or other food.
